(19) **Europäisches Patentamt / European Patent Office / Office européen des brevets**

(11) **EP 2 681 549 B1**

(12) **EUROPEAN PATENT SPECIFICATION**

(45) Date of publication and mention of the grant of the patent:
**01.11.2017 Bulletin 2017/44**

(21) Application number: **12713317.1**

(22) Date of filing: **28.02.2012**

(51) Int Cl.:
**G01N 33/28** (2006.01)   **G01N 31/22** (2006.01)

(86) International application number:
**PCT/IB2012/050908**

(87) International publication number:
**WO 2012/117341 (07.09.2012 Gazette 2012/36)**

(54) **PROCESS FOR THE DETERMINATION OF THE TOTAL ACIDITY NUMBER (TAN) OF A MINERAL INSULATING OIL**

VERFAHREN ZUR BESTIMMUNG DER GESAMTSÄUREANZAHL (TAN) EINES MINERALISOLIERÖLS

PROCÉDÉ POUR LA DÉTERMINATION DE L'INDICE D'ACIDITÉ TOTAL (TAN) D'UNE HUILE ISOLANTE MINÉRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2011 IT TO20110170**

(43) Date of publication of application:
**08.01.2014 Bulletin 2014/02**

(73) Proprietor: **Sea Marconi Technologies Di Vander Tumiatti S.a.s.**
**10093 Collegno (Torino) (IT)**

(72) Inventors:
- **ROGGERO, Carlo Maria**
  **I-10144 Torino (IT)**
- **DI CARLO, Stefano**
  **I-10129 Torino (IT)**
- **TUMIATTI, Vander**
  **I-10090 Rosta (Torino) (IT)**
- **TUMIATTI, Michela**
  **I-10143 Torino (IT)**

(74) Representative: **Gerbino, Angelo et al**
**Jacobacci & Partners S.p.A.**
**Corso Emilia 8**
**10152 Torino (IT)**

(56) References cited:

US-A- 5 800 782    US-A- 6 027 940

- Y TUR'YAN: "pH-Metric determination of acid numbers in petroleum oils without titration", TALANTA, vol. 47, no. 1, 1 September 1998 (1998-09-01), pages 53-58, XP55026970, ISSN: 0039-9140, DOI: 10.1016/S0039-9140(98)00031-9
- L. SZEBELLÉDY ET AL: "Mikromethode zur Bestimmung der Säurezahl des Ricinusöles", ANALYTICAL AND BIOANALYTICAL CHEMISTRY, vol. 107, no. 7-8, 1 July 1936 (1936-07-01), pages 269-276, XP055005894, ISSN: 1618-2642, DOI: 10.1007/BF01391596
- E STROCHKOVA: "pH-Metric method for acid number determination in hydraulic oils without titration", TALANTA, vol. 50, no. 5, 1 December 1999 (1999-12-01), pages 1135-1139, XP55026972, ISSN: 0039-9140, DOI: 10.1016/S0039-9140(99)00192-7
- Reinhard Matissek ET AL: "Fette und Fettbegleitstoffe" In: "Lebensmittelanalytik", 18 November 2009 (2009-11-18), Springer Berlin Heidelberg, Berlin, Heidelberg, XP055142277, ISSN: 0937-7433 ISBN: 978-3-54-092205-6 pages 47-48, DOI: 10.1007/978-3-540-92205-6_2,
- "Insulating liquids - Determination of acidity - Part 2: Colourimetric titration", IEC 62021-2:2007, IEC, 3, RUE DE VAREMBÉ, PO BOX 131, CH-1211 GENEVA 20, SWITZERLAND, 15 January 2007 (2007-01-15), pages 1-32, XP082004026, [retrieved on 2007-01-15]

EP 2 681 549 B1

## Description

This invention relates to a process for the determination of the total acidity number (TAN) in mineral insulating oils, in compliance with the Best Available Techniques (BAT) and Best Environmental Practices (BEP) guidelines for Smart Field Test(SFT).

The total acidity number (TAN) is a priority property for the evaluation of the characteristics of oils and organic liquids in general in compliance with the requisites prescribed by the norms of the sector. For example, in the case of mineral insulating oils for transformers, the TAN is the measure of the quantity of acids present, symptomatic of the phenomena of oxidation accelerating the functional degradation and/or the corrosion of components and/or material they are in contact with. This property is prescribed for the periodic monitoring to be carried out during the life cycle of the oil and the equipment in compliance with the technical norms of the sector, such as for example IEC 60296 and IEC 60422 and the laboratory method such, for example, IEC 62021.

Instead, in the case of vegetable oils, such as for example olive oil, the total acidity is the measure of the quantity of free fatty acids. This parameter affects the quality of the oil in terms of organoleptic and nutritive characteristics and is correlatable to the origin and the extraction and production methodology of the oil and/or its derivatives (natural esters).

In the case of derivatives from petroleum oil (crude oil), the Total Acidity Number (TAN) provides the information relative to the quantity of carboxylic compounds, normally of a naphtenic nature, present in the hydrocarbon fraction that during the distillation operations, for example, cause corrosion damages to pipes, transfer lines and distillation columns under vacuum. Instead, in insulating liquids, a high acidity value is symptomatic of factors of degradation correlatable to phenomena of thermal oxidation and the loss of one or more functional properties (for example dissipation factor - DF; interfacial tension - IFT etc.). Consequently, a specific periodic monitoring of the TAN (group 1, category A with minimum frequency once every 12 months for strategic transformers) is prescribed in compliance with the norms of the sector (for example IEC 60422).

Several norms exist prescribing the method of quantification of the TAN in relation to the limit of use and the field of application. In the case of petroleum derivatives there are, for example, norm ASTM D664 (Standard Test Method for Acid Number of Petroleum Products by Potentiometric Titration), IEC 62021 (Part 1 and 2 for insulating liquids and part 3 for non-insulating liquids. Potentiometric and Colourimetric titrations) and norm ASTM D974 (Standard Test Method for Acid and Base Number by Colour-Indicator Titration). Some kits are offered on the market for the in-field and laboratory determination of the TAN. For example:

- Kit Dexsil® TitraLube for the determination of the TAN on lubricating oils. This kit is described in patent US-5 800 782 (1/09/1998)
- Kit by Kittiwake total acid number (TAN) test.

Both the technical norms mentioned above and the current kits for the determination of the TAN available on the market (example Dexsil and Kittiwake) have some critical factors.

Critical factor I - Relatively high investments in laboratory equipment and infrastructure and the education of chemist personnel for the determination of the TAN in compliance with norms ASTM D664 and IEC 6202. They require the use of expensive and bulky laboratory equipment (for example Potentiometric Titration devices, autosamplers, electrodes).

Critical factor II - Skilled chemistry personnel, if a relatively significant number of samples must be analysed, in compliance with methods ASTM D664 and IEC 62021, which are hardly applicable in the field, especially by persons without chemical formation.

Critical factor III - The response time of the laboratory methods ASTM D664 and IEC 62021 may be incompatible with the needs for a fast check during in-fieldd operations. "Field tests" (for example, in the case of treatment of the oil of the transformers, the physical-chemical depolarisation or reclamation, in compliance with norm IEC 60422).

Critical factor IV - Relatively high volumes of samples and correlated wastes produced by the implementation of norms ASTM D664 and IEC 62021 (10 g sample, 50 ml of solvent, for example, for a total of at least 100 g of waste to be disposed of for each sample processed).

Critical factor V - Limit of application on dark oils of norm ASTM D974.

Critical factor VI - Seven operational phases according to the procedure of kit Dexsil®, including also the transfer of the solution to be titrated from one container to another with the possibility of some loss, thus diminishing the precision in terms of repeatability.

Critical factor VII - The limit of quantification of the kit Dexsil for the TAN is equivalent to 0.1 mg KOH/g oil, thus is not complying with the requisites prescribed by norms IEC 60296 (for new mineral insulating oils) and IEC 62021 for the determination of the TAN on minerals insulating oils, the limit of which is prescribed as 0,01 mg KOH/g oil.

Critical factor VIII - The limit of quantification and the accuracy of the kit Kittiwake for the TAN is not complying with the requisites prescribed by norms IEC 60296 (for new mineral insulating oils) and IEC 62021 for the determination of the TAN on minerals insulating oils. In particular, the Kittiwake method determines the TAN between 0 and 6 (mg KOH/g oil), but with an accuracy $\pm$ 0,3.

Critical factor IX - Conservation of the titrant agent from the deactivating action of the carbon dioxide - $CO_2$ (carbonation) determining the progressive loss of the titre with the subsequent increment in the measurement error of the TAN.

Critical Factor X - Relatively high investments and education costs for the laboratory personnel for the determination of the TAN in the case of olive oils for which the Regulation (EC) no. 1989/2003 and subsequent modifications applies. According to this regulation the determination is carried out by standard laboratory equipment, for example burettes that must be handled carefully by specialised personnel in equipped laboratories.

Scope of this invention is to provide a process for the quantitative colorimetric determination of the total acidity number (TAN) of an organic liquid, capable of overcoming the critical factors listed above.

This scope is achieved thanks to a process having the characteristics listed in one or more of the claims that follow.

Within this description, "titrant solutions" is intended a solution with a known concentration of a given base chemical species (in particular potassium hydroxide) that dosed through a micro-dispenser allows quantifying the concentration of the acid compound/s. Such pre-dosed solution is advantageously prepared in an inert atmosphere (argon, nitrogen, helium) and kept in a gas-sealed container for the protection of the deactivating action provided by the atmosphere (carbonation action of the base by carbon dioxide and conversion in alkali carbonate - $M_2CO_3$).

For "micro-dosing" is intended a mechanical/electronic device for the precise dosing of discrete and variable aliquots of titrant solution.

The organic liquids on which the process of the invention can be carried out are mineral based insulating oils.

The process of the invention allows the extraction of the acidic species from the organic liquid by means of an appropriate hydro-alcoholic solution - for example on the basis of methyl alcohol, ethyl alcohol, isopropyl alcohol. It provides the quantification by a colorimetric way of the acidic components by means of an acid-base titration. All this can be advantageously carried out on small volumes and inside a sole single use container, using, as dosing instrument, a micro- dispenser, instead of bulky and fragile burettes and laboratory glassware, requiring accurate washing and high operational costs.

On its side, the titrant solution can be specifically prepared in appropriate sealed containers in which an oxygen and carbon dioxide -free atmosphere is ensured.

Thanks to the process of the invention, the TAN can be determined with high levels of repeatability and reproducibility both in the field and in the laboratory by non-specialised personnel without requiring expensive and bulky dedicated equipment. Thanks to it, it is possible to measure values of acidity comprised between 0,010 and 2,00 mg KOH/g oil for insulating oils. Such wide range of measurement is not attainable by other kits for the determination of the TAN, currently offered on the market.

Thus, the process of the invention offers a simple, economic and fast response to all the application scenarios for the determination of the TAN in compliance with the guidelines of the Best Available Techniques (BAT) and Best Environmental Practices (BEP) for Smart Field Test(SFT).

In particular, the process of the invention allows the reduction of the sample volume and the quantity of reagents required for the determination of the TAN. Thus the environmental impact is minimised, with a reduction of up to 10 times of the volume of wastes produced, with respect to the traditional methods of analysis. With regard to the norms mentioned above, the quantities of sample, extraction solution and titrant solution are, in fact, much lower.

For example, for the determination according to IEC 62021 a TAN of 0,1 mg/g on an insulating oil are required 10 g of sample, 48 ml of isopropanol and 178 $\mu$l of titrant solution of KOH in isopropanol (0,1 M). On the contrary, according to this invention, only 4,5 g of oil, 2,5 ml of extracting solution and 80 $\mu$l of titrant solution of KOH in isopropanol (0,1 M) are usually required.

Additional advantages and features of this invention would be evident from the detailed description that follows, provided as a not limiting example with reference to the enclosed drawings, in which:

figure 1 illustrates the measuring container used by the process of the invention, and

Figure 2 is a graph illustrating the nearly null discrepancy between the results of the determination of TAN carried out on samples of mineral insulating oil with norm IEC 62021 (dotted line) and the method subject of this invention (continuous line).

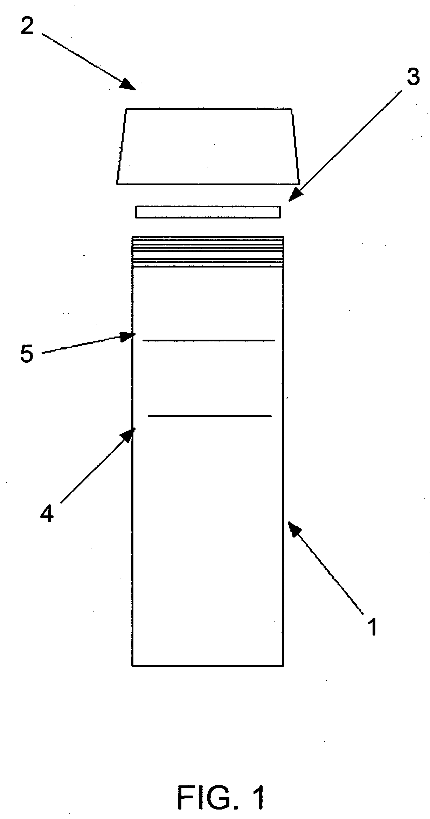

In figure 1, reference number 1 indicates the transparent measurement container, reference number 2 a screw type plug for the container, reference number 3 a sealing ring placed between plug 2 and container 1, reference number 4 a first mark indicating the level that must be reached in container 1 by the organic liquid and reference number 5 a second mark indicating the level that must be reached in container 1 by the extraction solution.

The process of this invention comprises the pouring, in container 1, of a liquid sample of unknown TAN until the first mark 4 is reached, then of an amount of a suitable hydro-alcoholic solution for extracting the acid fraction of said sample, for example on the basis of methyl alcohol, ethyl alcohol, isopropyl alcohol, until the second mark 5 is reached.

Then, container 1 is sealed by plug 2, the sealing of which is ensured by ring 3, shaked and allowed to rest until the separation between the sample and the extract phases occurs. Afterwards, a titrant solution of KOH is dosed into the extract phase in container 1, until an acid-based indicator present therein changes colour and finally the TAN is calculated.

For mineral insulating oils, the TAN can be calculated on the basis of the formula:

$$\mathrm{TAN\ (mg_{KOH}/g_{Oil})} = (V \times 56{,}1 \times M)/m \qquad (1)$$

where V is the volume of dosed titrant solution expressed in ml, 56,1 is the molecular weight of KOH, M is the titre of the KOH solution expressed in moles per litre and m is the mass of the sample expressed in g.

In the case of dark mineral insulating oils it has been surprisingly found that the extracting solution is not affected by the dark component of the oil. Thus the analysis of oxidised oils, which are normally those with higher acidity, is not subjected to significant interferences and good precision in the determination of the TAN is insured.

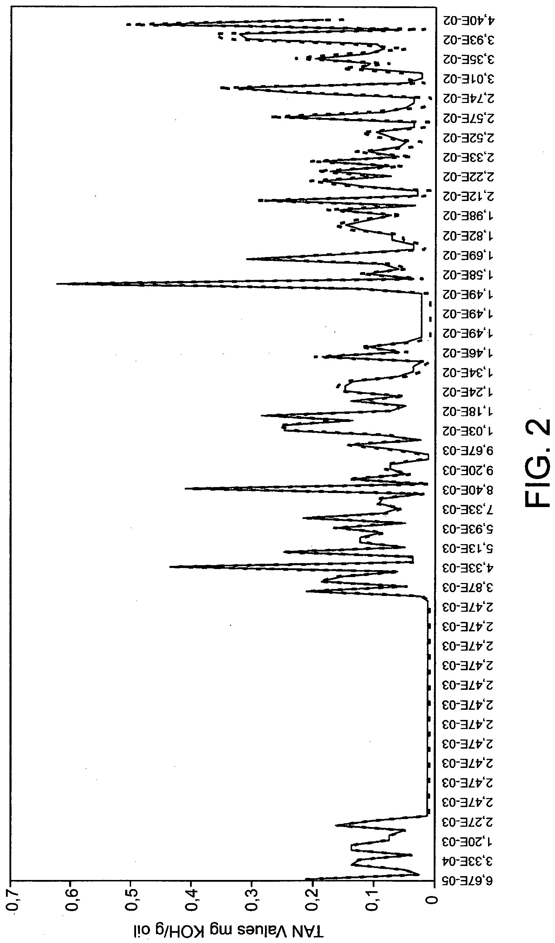

Moreover, it has been found that the process of this invention provides results of TAN equivalent to those obtained with the known methods mentioned above (IEC 62021), as it can be deduced from figure 2, where the differences in the absolute values between the TAN obtained by the method described in norm IEC 62021 and the TAN calculated with the process of this invention, on a series of samples of oil, examined are shown. In particular, it is noted that the difference found between the two methods is comprised between 0 and 0,04.

The following examples provided as non limiting title describe further the application of the process of the invention.

Example 1

The measuring container has been filled up to the first mark with mineral insulating oil (clear, colour: 0,5 according to ASTM D1500) with a TAN equivalent to 0,010 $mg_{KOH}/g_{Oil}$ previously measured in accordance with norm IEC 62021. The extracting solution, with the indicator already dissolved, has been added up to the second mark. The container has been plugged and vigorously shaken for a few seconds. The separation of the oleous phase from the extracted one, accumulated in the upper part of the container, was awaited. Using a micro-dosing dispenser, doses of alcoholic solution of KOH have been added until colour changing. The value of acidity expressed as $mg_{KOH}/g_{Oil}$ was 0,010.

Example 2

The measuring container has been filled up to the first mark with mineral insulating oil (clear, colour: 0,5 according to ASTM D1500) with a TAN equivalent to 0,033 $mg_{KOH}/g_{Oil}$ previously measured in accordance with norm IEC 62021. The extracting solution with the indicator already dissolved, has been added up to the second mark. The container has been plugged and vigorously shaken for a few seconds. The separation of the oleous phase from the extracted one, accumulated in the upper part of the container, was awaited. Using a micro-dosing dispenser, doses of alcoholic solution of KOH have been added until colour changing. The value of acidity expressed as $mg_{KOH}/g_{Oil}$ was 0,037.

Example 3

The measuring container has been filled up to the first mark with mineral insulating oil (dark yellow, colour: 2 according to ASTM D1500) with a TAN equivalent to 0,209 $mg_{KOH}/g_{Oil}$ previously measured in accordance with norm IEC 62021. The extracting solution with the indicator already dissolved, has been added up to the second mark. The container has been plugged and vigorously shacken for a few seconds. The separation of the oleous phase from the extracted one, accumulated in the upper part of the container was awaited. Using a micro-dosing dispenser, doses of alcoholic solution of KOH have been added until colour changing. The value of acidity expressed as $mg_{KOH}/g_{Oil}$ was 0,194.

<u>Example 4</u>

The measuring container has been filled up to the first mark with mineral insulating oil (very dark, colour: 7 according to ASTM D1500) with a TAN equivalent to 0,608 $mg_{KOH}/g_{Oil}$ previously measured in accordance with norm IEC 62021. The extracting solution with the indicator already dissolved, has been added up to the second mark. The container has been plugged and vigorously shaken for a few seconds. the separation of the oleous phase from the extracted one, accumulated in the upper part of the container was awaited. Using a micro-dosing dispenser, doses of alcoholic solution of KOH have been added until colour changing. The value of acidity expressed as $mg_{KOH}/g_{Oil}$ was 0,623.

**Claims**

1. Method for determining the total acid number (TAN) of an organic liquid, which is a mineral insulating oil, comprising the steps of:

   - pouring in a single use container a sample of said liquid having a mass comprised between 1 and 10 g and a volume comprised between 1 and 10 ml of a solution suitable for extracting the acid fraction of said sample and sealing the container;
   - stirring the content of the container and waiting for the separation from the sample of an extract phase;
   - dosing in the extract phase in said container a titrant solution of KOH contained in a micro-dispenser until an acid-base indicator present therein changes color, so that the titration step takes place in the same container wherein the sample was poured; and
   - calculating the TAN on the basis of the volume of dosed titrant solution according to the formula:

$$ \text{TAN } (mg_{KOH}/g_{oil}) = (V \times 56,1 \times M)/m \qquad (1) $$

   wherein V is the volume of dosed titrant solution expressed in ml, 56,1 is the molecular weight of KOH, M is the titre of the KOH solution expressed in moles per litre and m is the mass of the sample expressed in g.

2. Method according to claim 1 , wherein said sample has a mass of 4.5 g, and said extracting solution has a volume of 2 ml.

3. Method according to any one of the previous claims, wherein said extracting solution is a hydro-alcoholic solution.

4. Method according to claim 3, wherein said extracting solution is on the basis of methyl alcohol, ethyl alcohol and/or isopropyl alcohol.

5. Method according to any one of the previous claims, wherein said titrant solution is dosed by micro-dosing device in discrete amounts comprised in a range between 1 and 1000 $\mu$l.

6. Method according to any one of the previous claims, wherein said titrant solution is taken from a container insulated from the external ambient and having internally an atmosphere of inert gas, in particular argon, nitrogen or helium.

**Patentansprüche**

1. Verfahren zum Bestimmen der Gesamtsäurezahl (TAN) einer organischen Flüssigkeit, die ein mineralisches Isolieröl ist, anhand der folgenden Arbeitsschritte;

   - Gießen in einen Einweg-Behälter einer Probe der besagten Flüssigkeit mit einer Masse zwischen 1 und 10 g und einem Volumen zwischen 1 und 10 ml einer Lösung, die zum Extrahieren des Säureanteils der genannten Probe geeignet ist; Abdichten des Containers;
- Verrühren des Behälterinhalts und Warten, dass sich von der Probe eine Extraktphase trennt;
- Zudosierung in die Extraktphase des besagten Behälters einer in einem Micro-Dispenser enthaltenen KOH-Titrierlösung, bis ein darin enthaltener Säure-Base-Indikator seine Farbe ändert, so dass der Titrierschritt in demselben Behälter stattfindet, in den die Probe gegossen wurde; und
- Berechnung der Gesamtsäurezahl (TAN) auf der Grundlage des Volumens der dosierten Titrierlösung nach der Formel:

$$TAN\ (mg_{KOH}/g_{Öl}) = (V \times 56{,}1 \times M)/m \qquad (1)$$

wobei V das Volumen der dosierten Titrierlösung in ml, 56,1 das Molekulargewicht des KOH, M der Titer der KOH-Lösung in Mol/l und m die Masse der Probe in g ist.

2. Verfahren nach Anspruch 1, wobei besagte Probe eine Masse von 4,5 g und besagte Extrahierlösung ein Volumen von 2 ml hat.

3. Verfahren nach einem der vorangegangenen Ansprüche, wobei besagte Extrahierlösung eine hydroalkoholische Lösung ist.

4. Verfahren nach Anspruch 3, wobei besagte Extrahierlösung auf Methylalkohol, Ethylalkohol und/oder Isopropylalkohol basiert.

5. Verfahren nach einem der vorangegangenen Ansprüche, wobei besagte Titrierlösung anhand eines Mikrodosiergeräts in einer diskreten Menge zwischen 1 und 1000 $\mu$l dosiert wird.

6. Verfahren nach einem der vorangegangenen Ansprüche, wobei besagte Titrierlösung einem von der externen Umgebung isolierten Behälter entnommen wird und in interner Umgebung eine Inertgasatmosphäre aufweist, die hauptsächlich aus Argon, Stickstoff oder Helium besteht.

## Revendications

1. Procédé de détermination de l'indice d'acidité total (TAN) d'un liquide organique, en l'occurrence une huile minérale isolante, y compris les étapes suivantes ;

- verser dans un récipient à usage unique un échantillon du liquide ayant une masse comprise entre 1 et 10 g et un volume compris entre 1 et 10 ml d'une solution appropriée pour l'extraction de la fraction acide de l'échantillon, puis sceller le récipient ;
- mélanger le contenu du récipient et attendre la séparation de phase de l'échantillon ;
- doser dans la fraction extraite du récipient une solution titrante de KOH contenue dans un mini-distributeur jusqu'à ce que l'indicateur acido-basique qui s'y trouve change de couleur, de manière à ce que l'étape de titrage ait lieu dans le même récipient dans lequel l'échantillon a été versé ; et
- calculer le TAN sur la base du volume de solution titrante dosée selon la formule :

$$TAN\ (mg_{KOH}/g_{huile}) = (V \times 56{,}1 \times M)/m \qquad (1)$$

où V est le volume de la solution titrante dosée exprimé en ml, 56,1 est le poids moléculaire du KOH, M est le titre de la solution de KOH exprimé en moles par litre et m est la masse de l'échantillon exprimée en g.

2. Procédé selon la revendication 1, dans lequel ledit échantillon a une masse de 4,5 g, et ladite solution extraite a un volume de 2 ml.

3. Procéder selon l'une quelconque des revendications précédentes, dans lequel ladite solution extraite est une solution hydro-alcoolique.

**4.** Procédé selon la revendication 3, dans lequel ladite solution extraite est à base d'alcool méthylique, d'alcool éthylique et/ou d'alcool isopropylique.

**5.** Procédé selon l'une quelconque des revendications précédentes, dans lequel ladite solution titrante est dosée par un dispositif de microdosage en faible quantité comprise entre 1 et 1000 μl.

**6.** Procéder selon l'une quelconque des revendications précédentes, dans lequel ladite solution titrante est tirée d'un récipient isolé de l'environnement externe et ayant à l'intérieur une atmosphère de gaz inerte, en particulier argon, azote ou hélium.

EP 2 681 549 B1

FIG. 1

8

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5800782 A